(19) Europäisches Patentamt
European Patent Office
Office européen des brevets

(11) EP 0 708 093 B1

(12) # EUROPÄISCHE PATENTSCHRIFT

(45) Veröffentlichungstag und Bekanntmachung des Hinweises auf die Patenterteilung:
**17.01.2001 Patentblatt 2001/03**

(51) Int Cl.[7]: **C07D 241/44**, A61K 31/495

(21) Anmeldenummer: **95116094.4**

(22) Anmeldetag: **12.10.1995**

(54) **Chinoxaline, Verfahren zu ihrer Herstellung und ihre Verwendung**

Quinoxalines, process for their preparation, and their use

Quinoxalines, procédé pour leur préparation et leur application

(84) Benannte Vertragsstaaten:
**AT BE CH DE DK ES FR GB GR IE IT LI LU NL PT SE**

(30) Priorität: **19.10.1994 DE 4437406**

(43) Veröffentlichungstag der Anmeldung:
**24.04.1996 Patentblatt 1996/17**

(73) Patentinhaber: **Aventis Pharma Deutschland GmbH**
**65929 Frankfurt am Main (DE)**

(72) Erfinder:
- **Rösner, Manfred, Dr.**
  **D-65817 Eppstein (DE)**
- **Billhardt-Troughton, Uta-Maria, Dr.**
  **Raleigh, N.C. 27606 (US)**
- **Kirsch, Reinhard, Dr.**
  **D-38100 Braunschweig (DE)**
- **Kleim, Jörg-Peter, Dr.**
  **D-65779 Kelkheim (DE)**
- **Meichsner, Christoph, Dr.**
  **D-65835 Liederbach (DE)**
- **Riess, Günther, Dr.**
  **D-65795 Hattersheim (DE)**
- **Winkler, Irvin, Dr.**
  **D-65835 Liederbach (DE)**

(56) Entgegenhaltungen:
**EP-A- 0 509 398**

- **BIOCHEMICAL AND BIOPHYSICAL RESEARCH COMMUNICATIONS, Bd.201, Nr.3, 30. Juni 1994 Seiten 1305 - 1312 J. BALZARINI ET AL. 'Sensitivity of ...'**
- **JOURNAL OF VIROLOGY, Bd.68, Nr.12, 4 Seiten 7986 - 7992 J. BALZARINI ET AL. 'Resistance Pattern of Human ...'**

Bemerkungen:
Die Akte enthält technische Angaben, die nach dem Eingang der Anmeldung eingereicht wurden und die nicht in dieser Patentschrift enthalten sind.

EP 0 708 093 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Gegenstand der vorliegenden Erfindung sind Chinoxaline, Verfahren zu ihrer Herstellung und ihre Verwendung als Virustatika, insbesondere zur Behandlung von Infektionen mit dem "Human Immunodeficiency Virus" (HIV). In der europäischen Patentanmeldung EP-509398-A sind Chinoxalinderivate für diesen Anwendungsbereich beschrieben.
Es wurde nun überraschenderweise gefunden, daß eine Gruppe speziell substituierter Chinoxaline der Formel I,

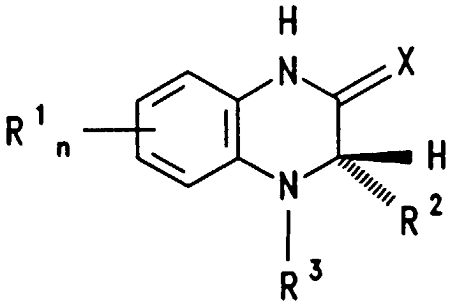

(I)

sowie deren tautomere Formen der allgemeinen Formel Ia

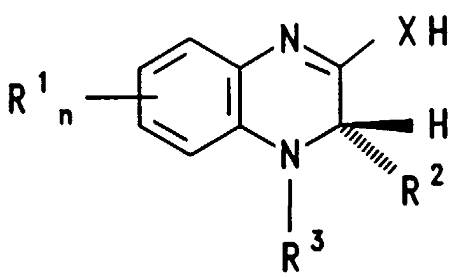

(Ia)

sowie deren physiologisch verträgliche Salze
oder Prodrugs eine antivirale Wirkung insbesondere gegen Retroviren, wie zum Beispiel das 'Human Immunodeficiency Virus' (HIV) aufweisen.
In den erfindungsgemäßen Verbindungen der Formel I bzw. Ia bedeuten:

1) n gleich null oder eins,

$R^1$ Fluor, Chlor, Hydroxy, $C_1$-$C_3$-Alkoxy,

$R^2$ $C_1$-$C_4$-Alkyl, gegebenenfalls substituiert durch Hydroxy, $C_1$-$C_4$-Alkoxy, oder $C_1$-$C_4$-Alkylthio,

$R^3$ $C_1$-$C_4$-Alkyloxycarbonyl oder $C_2$-$C_4$-Alkenyloxycarbonyl,

X bedeutet Sauerstoff oder Schwefel.

In einer nochmals bevorzugten Gruppe von Verbindungen der Formel I bzw. Ia bedeuten:

3) n gleich null oder eins,

$R^1$ Fluor, Chlor, Methoxy, Ethoxy, Propoxy,

$R^2$ Methylthiomethyl, Ethyl, Propyl, $C_1$-$C_2$-Alkyl substituiert durch Hydroxy oder $C_1$-$C_4$-Alkoxy,

$R^3$ $C_1$-$C_4$-Alkyloxycarbonyl oder $C_2$-$C_4$-Alkenyloxycarbonyl,

X bedeutet Sauerstoff oder Schwefel.

Eine ganz besondere Bedeutung haben diejenigen Verbindungen der Formel I oder Ia wie oben beschrieben, worin die genannten Substituenten bedeuten:

4) n gleich null oder eins,

$R^1$ Fluor, Chlor, Methoxy, Ethoxy,

$R^2$ Methylthiomethyl, Ethyl, Propyl, $C_1$-$C_2$-Alkyl substituiert durch Hydroxy oder $C_1$-$C_4$-Alkoxy,

$R^3$ $C_1$-$C_4$-Alkyloxycarbonyl oder $C_2$-$C_4$-Alkenyloxycarbonyl,

X bedeutet Sauerstoff oder Schwefel.

Eine ganz besondere Bedeutung besitzt die Verbindung S-4-Isopropoxycarbonyl-6-methoxy-3-(methylthiomethyl)-3,4-dihydrochinoxalin-2(1H)-thion (Bsp. 85).
Die Verbindungen der Formeln I und Ia besitzen ein asymmetrisches Kohlenstoffatom.
Es wurde nun überraschenderweise gefunden, daß die erfindungsgemäßen Verbindungen eine unerwartet deutlich gesteigerte antivirale Wirklung besitzen. Es wurde ferner gefunden, daß die reinen Enantiomeren deutlich leichter löslich sind als die zugehörigen racemischen Verbindungen. Diese liegen als echte Racemate, d.h. als 1: 1-Verbindungen der beiden Enantiomeren, mit individuellen physikalischen Eigenschaften vor.
Als Folge davon werden die reinen Enantiomeren in Tierversuchen nach oraler Gabe deutlich besser resorbiert. Dies ist eine wichtige Voraussetzung für die Entwicklung eines neuen Arzneimittels.
Es ist allgemein bekannt, daß zur Erzielung einer möglichst starken pharmakologischen oder chemotherapeutischen Wirkung hohe erreichbare Blutspiegel von besonderer Wichtigkeit sind.
In Anbetracht der Tatsache, daß mit vielen potentiellen Virustatika gegen HIV aufgrund ihrer niedrigen Bioverfügbarkeit nach oraler Gabe keine ausreichenden Blutspiegel für die Unterdrückung der Virusreplikation erreicht werden konnten, stellen die erfindungsgemäßen Verbindungen überlegen wirksame antivirale Mittel und somit einen therapeutischen Fortschritt dar.
Die reinen Enantiomeren der Verbindungen der Formeln I und Ia lassen sich durch bekannte Methoden oder in Analogie zu bekannten Methoden direkt herstellen oder nachträglich trennen.
Die Verbindungen der Formeln I und Ia lassen sich nach bekannten Methoden oder Modifikationen derselben herstellen (s. z.B. EP-509398-A, Rodd's Chemistry of Carbon Compounds, S. Coffey, M. F. Ansell (Herausgeber); Elsevier, Amsterdam, 1989; Vol. IV Teil IJ, S. 301 bis 311. Heterocyclic Compounds, R. C. Elderfield (Herausgeber); Wiley, New York, 1957; Vol. 6, S. 491 bis 495).
Zum Gegenstand der vorliegenden Erfindung gehört weiterhin ein Verfahren zur Herstellung von Verbindungen der Formeln I und Ia wie oben unter 1) bis 4) erläutert, dadurch gekennzeichnet, daß man

A) zur Herstellung von Verbindungen der Formel I mit X gleich Sauerstoff und den Resten $R^1$, $R^2$ und $R^3$ wie in unter 1) bis 4) definiert eine Verbindung der Formel II,

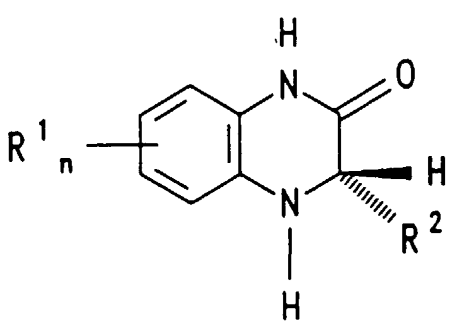

(II)

wobei für $R^1$ und $R^2$ die in unter 1) bis 4) genannten Definitionen gelten, mit einer Verbindung der Formel III,

$$R^3\text{-}Z \qquad (III)$$

wobei $R^3$ die oben unter 1) bis 4) genannten Bedeutungen hat und Z eine Abgangsgruppe wie zum Beispiel Chlor ist, umsetzt oder daß man

B) Verbindungen der Formel I, mit X gleich Schwefel und $R^1$, $R^2$ und $R^3$ wie unter 1) bis 4) definiert herstellt durch Reaktion einer Verbindung der Formel I, wobei X Sauerstoff ist und für $R^1$, $R^2$ und $R^3$ die unter 1) bis 4) genannten Definitionen gelten, mit einem Schwefelungsreagenz.

Bei der obengenannten Methode A) erfolgt die Umsetzung vorzugsweise mit einem Halogenameisensäurealkyl- oder alkenylester, einem Dialkyl- oder Dialkenylcarbonat oder einem Pyrokohlensäuredialkyl- oder alkenylester. Der Substituent Z in der Formel III ist demnach eine geeignete Abgangsgruppe, wie z.B. Chlor, Brom oder Jod, ein Alkoxy- oder Alkenyloxyrest oder ein Alkoxy- oder Alkenyloxycarbonyloxy-Gruppe. Vorzugsweise ist Z gleich Chlor.

Die Umsetzung wird zweckmäßigerweise in einem inerten Lösungsmittel durchgeführt. Geeignet sind z.B. aromatische Kohlenwasserstoffe wie Toluol oder Xylol, niedere Akohole wie Methanol, Ethanol oder 1-Butanol, Ether wie Tetrahydrofuran oder Glycoldimethylether, dipolar aprotische Lösungsmittel wie N,N-Dimethylformamid, N-Methyl-2-pyrrolidon, Acetonitril, Nitrobenzol, Dimethylsulfoxid oder Gemische dieser Lösungsmittel. Auch Zweiphasensysteme mit wäßrigen Lösungen von Basen in Gegenwart eines Phasentransferkatalysators, wie z.B. Benzyltriethylammoniumchlorid, sind möglich.
Die Anwesenheit einer geeigneten Base z.B. eines Alkali- oder Erdalkalimetallcarbonats oder -hydrogencarbonats wie Natriumcarbonat, Calciumcarbonat oder Natriumbicarbonat, eines Alkali- oder Erdalkalihydroxids wie Kaliumhydroxid oder Bariumhydroxid, eines Alkoholats wie Natriumethanolat oder Kalium-tert.-butylat, einer lithiumorganischen Verbindung wie Butyllithium oder Lithiumdiisopropylamid, eines Alkali- oder Erdalkalihydrids wie Natriumhydrid oder Calciumhydrid, ein Alkalifluorid wie Kaliumfluorid oder einer organischen Base wie Triethylamin, Pyridin, 4-Methylpyridin oder 4-(Dimethylamino)pyridin zum Auffangen der bei der Reaktion freiwerdenden Säure kann nützlich sein.
In manchen Fällen ist der Zusatz eines Jodsalzes, z.B. Kaliumjodid, angebracht. Die Reaktion wird gewöhnlich bei Temperaturen zwischen -10 und 160°C durchgeführt, vorzugsweise bei Raumtemperatur.

Für diese Umsetzung müssen etwaige nucleophile Substituenten wie z.B. Hydroxy-, Mercapto- oder Aminogruppen mit Ausnahme der 4-Position in Verbindungen der Formel II vor Durchführung der Reaktion in geeigneter Weise derivatisiert oder mit wieder abspaltbaren gebräuchlichen Schutzgruppen wie z.B. Acetyl, Benzyl, Trityl, Tetrahydropyranyl oder tert.-Butoxycarbonyl versehen werden.

Für die Umsetzung wie zuvor unter B) beschrieben, wird vorzugsweise als Schwefelungsreagenz 2,4-Bis (4-methoxyphenyl)-1,3-dithia-2,4-diphosphetan-2,4-disulfid (Lawessons Reagenz), Bis(tricyclohexylzinn)sulfid, Bis (tri-n-butylzinn)sulfid, Bis(triphenylzinn)sulfid, Bis(trimethylsilyl)sulfid oder Phosphorpentasulfid verwendet.
Die Reaktion wird zweckmäßigerweise in einem organischen Lösungsmittel oder einem Lösungsmittelgemisch, bei -10 bis 120°C, bevorzugt bei Raumtemperatur bis 60°C und möglichst unter wasserfreien Bedingungen durchgeführt. Geeignet sind z.B. Schwefelkohlenstoff, Toluol, Xylol, Pyridin, Dichlormethan, 1,2-Dichlorethan, Tetrahydrofuran, Essigsäureethylester oder Essigsäurebutylester. Bei Verwendung der erwähnten Zinn- oder Silylsulfide ist es angebracht, die Schwefelungsreaktion in Gegenwart einer Lewissäure wie Bortrichlorid durchzuführen.
Die Gegenwart einer Carbonylgruppe im Rest $R^3$ in den Verbindungen der Formel I stört dabei wegen ihrer geringeren Reaktivität nicht, so daß eine selektive Schwefelung möglich ist.

Die als Ausgangsmaterialien für die beschriebenen Synthesen benötigten Chinoxaline der allgemeinen Formel II sind literaturbekannt oder können nach bekannten Methoden, z.B. nach den in der europäischen Patentanmeldung EP-509398-A beschriebenen Verfahren hergestellt werden.

Weiterhin gehören zum Gegenstand der vorliegenden Erfindung die Verbindungen wie unter 1) bis 4) beschrieben als Arzneimittel, vorzugsweise zur Behandlung von Viruserkrankungen, insbesondere von Erkrankungen hervorgerufen durch das HIV.

Des weiteren betrifft die vorliegende Erfindung Arzneimittel mit einem Gehalt an mindestens einer erfindungsgemäßen Verbindung, sowie die Verwendung der genannten Verbindungen zur Herstellung von Arzneimitteln, vorzugsweise zur Behandlung von Viruserkrankungen, insbesondere zur Behandlung von Krankheiten, hervorgerufen durch das HIV.

Weiterhin gehört zum Gegenstand der vorliegenden Erfindung die Verwendung von Verbindungen der obengenannten Formel I bzw. Ia zur Herstellung von Arzneimitteln zur Behandlung von Viruserkrankungen.

Für diese Verwendung sind die oben unter 1) bis 4) genannten und erläuterten Verbindungen bevorzugt.

Die erfindungsgemäßen Arzneimittel können enteral (oral), parenteral (intravenös), rektal, subcutan, intramuskulär oder lokal (topisch) angewendet werden.

Sie können in Form von Lösungen, Pulvern (Tabletten, Kapseln einschließlich Microkapseln), Salben (Cremes oder Gele) oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage.

Als zweckmäßige Dosierung werden 0,1 bis 10, vorzugsweise 0,2 bis 8 mg/kg Körpergewicht ein oder mehrmals täglich verabreicht. Die verwendeten Dosierungseinheiten richten sich zweckmäßigerweise nach der jeweiligen Pharmakokinetik der verwendeten Substanz bzw. der verwendeten galenischen Zubereitung.

Die verwendete Dosierungseinheit der erfindungsgemäßen Verbindungen beträgt z.B. 1 bis 1500 mg, vorzugsweise 50 bis 500 mg.

Die erfindungsgemäßen Verbindungen können auch in Kombination mit anderen antiviralen Mitteln, wie z.B. Nucleosidanaloga, Proteaseinhibitoren oder Adsorptionsinhibitoren, Immunstimulantien, Interferonen, Interleukinen und Kolonie-stimulierenden Faktoren (z.B. GM-CSF, G-CSF, M-CSF), verabreicht werden.

Unter reinen Enantiomeren werden solche Verbindungen verstanden, in denen das Enantiomerenverhältnis mindestens 95:5, vorzugsweise mindestens 97:3 beträgt.

Durch die nachfolgenden Beispiele sowie durch den Inhalt der Patentansprüche wird die vorliegende Erfindung näher erläutert.

Beispiel 1:

N-(5-Fluor-2-nitrophenyl)-S-methyl-L-cystein

16,2 g (-)-S-Methyl-L-cystein (0,1 mol) werden in einem Gemisch von 120 ml Wasser und 120 ml Aceton im Vierhalskolben unter $N_2$ suspendiert. 30,4 ml (22,2 g) Triethylamin (0,22 mol) werden unter Rühren rasch zugegeben. In die entstehende gelbe Lösung werden unter weiterem Rühren 15,9 g 2,4-Difluornitrobenzol (0,1 mol) zugegeben.

Unter Rühren wird 7,5 Stunden zum Rückfluß erhitzt (orangefarbene Lösung), am Rotationsverdampfer das Aceton unter vermindertem Druck abgezogen und der wässrige Rückstand in einen Scheidetrichter überführt und 2x mit ca. 50 ml Methyl-tert.butylether (MTB-Ether) extrahiert. Dieser Extrakt besteht überwiegend aus 2,4-Difluornitrobenzol und wird verworfen.
Die wässrige Phase wird in einen Vierhalskolben überführt, mit 150 ml MTB-Ether versetzt und unter Kühlung (< 25°C) mit ca. 25 ml 38 %iger Schwefelsäure auf pH 1 gestellt. Es wird ausgerührt bis sich klare Phasen ergeben. Die Ether-Phase wird abgetrennt und die wässrige Phase noch einmal mit 50 ml MTB-Ether extrahiert.
Die Extrakte werden über Natriumsulfat getrocknet und am Rotationsverdampfer eingedampft. Ausbeute 27 g gelbes Öl, welches bald erstarrt, Schmp. 147° (aus Wasser/Methanol).
MS: Chemische Ionisation, $(M+H)^+$ = 275

| Analyse: | ber. | gef. |
|---|---|---|
| C | 43,8 % | 43,8 % |
| H | 4,0 % | 4,1 % |
| N | 10,2 % | 10,0 % |
| S | 11,7 % | 11,3 % |

Beispiel 2:

N-(5-Methoxy-2-nitrophenyl)-S-methyl-L-cystein

27 g N-(5-Fluor-2-nitrophenyl)-S-methyl-L-cystein, (0,1 mol) aus Beispiel 1 werden in einem Vierhalskolben in 150 ml absolutem Methanol gelöst und unter gutem Rühren, Argon und Kühlung mittels Eisbad innerhalb 20 Minuten portionsweise mit 14,4 g 95 %igem Natriummethylat (0,25 mol) versetzt. Unter Rühren wird 2 Stunden zum Rückfluß erhitzt. Nach DC-Kontrolle ist die Reaktion beendet.
Die Hauptmenge des Methanols wird am Rotationsverdampfer unter vermindertem Druck abgezogen. Der Rückstand wird mit 200 ml Eiswasser versetzt und mit ca. 25 ml 38 %iger Schwefelsäure auf pH 1 gestellt und mit 150 ml MTB-Ether ausgerührt. Die Ether-Phase wird abgetrennt und die wässrige Phase noch einmal mit 30 ml MTB-Ether extrahiert und unter vermindertem Druck einrotiert.
Ausbeute: 21,5 g braunrotes Öl, welches langsam kristallisiert.
MS: Chemische Ionisation, $(M + H)^+$ = 287
HPLC: 99,3 % R-Enantiomer

| Analyse: | ber. | gef. |
|---|---|---|
| C | 46,2 % | 47,3 % |
| H | 4,9 % | 5,6 % |
| N | 9,8 % | 9,1 % |
| S | 11,1 % | 10,6 % |

Beispiel 3:

R-6-Methoxy-3-(methylthiomethyl)-3,4-dihydrochinoxalin-2(1H)-on

20,7 g der Verbindung aus Beispiel 2 (0,065 mol) werden in 250 ml Methanolgelöst und unter Argon mit 0,5 ml Eisessig und ca. 20 g Raney-Nickel unter Normaldruck bei Raumtemperatur hydriert. Wenn nach DC kein Ausgangsmaterial mehr erkennbar ist, wird die Hydrierung beendet. Der Ansatz wird unter Stickstoffüberlagerung abgesaugt und mit 100 ml Methanol nachgewaschen.
Der Filterrückstand incl. Katalysator wird unter Stickstoffüberlagerung mit Dimethylformamid (DMF) bei 45 bis 50°C ausgerührt und anschließend erneut über eine Klärschicht abgesaugt. Die produkthaltige DMF-Lösung wird umgehend in 1 l Eiswasser, dem 2 g Ascorbinsäure als Antioxidans zugesetzt wurden, unter Rühren einlaufen gelassen. Das Produkt fällt dabei in Form hellgelber Kristalle an. Man saugt ab, wäscht mit ca. 2 l Wasser, anschließend mit 500 ml Ethanol und dann mit 300 ml Pentan nach und trocknet über Phosphorpentoxid.
Ausbeute 10,8 g, weitere 1,3 g können durch Einengen des Filtrats gewonnen werden. Schmp. 186 bis 187°C, gelbgräulicher Feststoff
$^1$H-NMR (200 MHz, $d_6$-DMSO): δ = 2,08 (s, 3 H, $SCH_3$), 2,75 ($dq_{AB}$, 2 H, -$CH_2$-S), 3,65 (s, 3 H, MeO), 3,95 (m, 1 H, CH), 6,05 (br, s, NH), 6,1 - 6,7 (m, 3 H, Aromaten), 10,15 (s, 1 H, Amid).
MS: Chemische Ionisation, $(M + H)^+$ = 239
HPLC: 97,5 % Reinheit, 98,2 % S-Enantiomer
Optische Rotation: $[\alpha]_D^{22}$ = -42° (c = 1 in Aceton)

| Analyse: | ber. | gef. |
|---|---|---|
| C | 55,5 % | 55,2 % |
| H | 5,9 % | 5,8 % |
| N | 11,8 % | 11,7 % |
| S | 13,4 % | 13,3 % |

In analoger Verfahrensweise erhält man:

Beispiel 4:

R-6-Ethoxy-3-(methylthiomethyl)-3,4-dihydrochinoxalin-2(1H)-on

Aus der Verbindung des Beispiels 1 mit Lithium-Ethylat in Ethanol und Reduktion und Ringschluß in Analogie zu Beispiel 2.
MS: Chemische Ionisation, $(M + H)^+$ = 253
$^1$H-NMR (200 MHz, $d_6$-DMSO): Ethoxygruppe δ = 1,27 (t, 3 H), 3,87 (q, 2 H)

Beispiel 5:

R-3-(Methylthiomethyl)-6-propoxy-3,4-dihydrochinoxalin-2(1H)-on

Aus der Verbindung des Beispiels 1 mit Natrium-Propylat in Propanol
Schmp. Harz, MS: Chemische Ionisation, $(M+H)^+$ = 267
$^1$H-NMR (200 MHz, $d_6$-DMSO): Propoxygruppe δ = 0,95 (t, 3 H), 1,67 (q, 2 H), 3,79 (t, 2 H)

Beispiel 6:

R-3-(Methylthiomethyl)-3,4-dihydrochinoxalin-2(1H)-on

Bei Verwendung von 2-Fluornitrobenzol anstelle von 2,4-Difluornitrobenzol in Beispiel 1
Schmp. 109°C, MS: Chemische Ionisation, $(M+H)^+$ = 208

Beispiel 7:

R-6-Fluor-3-(methylthiomethyl)-3,4-dihydrochinoxalin-2(1H)-on

Bei direkter Weiterverwendung der Verbindung aus Beispiel 1 in Reduktions- und Ringschlußreaktion gemäß Beispiel 3.
Schmp. 149°C, MS: Chemische Ionisation, $(M+H)^+$ = 243

Beispiel 8:

R-6-Chlor-3-(methylthiomethyl)-3,4-dihydrochinoxalin-2(1H)-on

Bei Verwendung von 2,4-Dichlornitrobenzol anstelle von 2,4-Difluornitrobenzol in Beispiel 1 und mit Natriumhydroxid und Glykolmonomethylether bei Rückflußtemperatur.
Schmp. 149°C, MS: Chemische Ionisation, $(M+H)^+$ = 243

In analoger Verfahrensweise zu den Beispielen 1 bis 8 können beispielsweise bei Verwendung anderer Aminosäuren die entsprechenden Verbindungen der Formel II, in denen der -$\alpha$-Substituent der eingesetzten Aminosäure zum Substituenten $R^2$ in Formel II wird, erhalten werden:

Tabelle 1

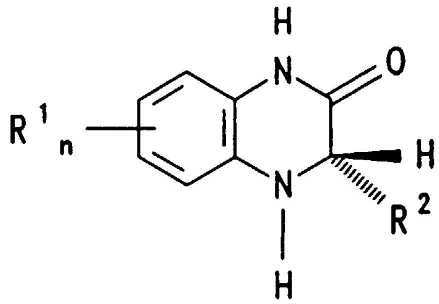

(II)

| Beispiel Nr. | $R^1_n$ | $R^2$ | Schmp. °C |
|---|---|---|---|
| 9 | H (n = 0) | $C_2H_5$ | Öl |
| 10 | H (n = 0) | $C_3H_7$ | Harz |
| 11 | H (n = 0) | $C_4H_9$ | Öl |
| 12 | H (n = 0) | $HO\text{-}CH_2$ | 82 |
| 13 | 6-Cl | $C_2H_5$ | 120 |
| 14 | 6-Cl | $C_3H_7$ | 75 - 77 |
| 15 | 6-Cl | $C_4H_9$ | Öl |
| 16 | 6-F | $C_2H_5$ | 93 |
| 17 | 6-F | $C_3H_7$ | Harz |
| 18 | 6-F | $HO\text{-}CH_2$ | 134 |
| 19 | 6-$CH_3$ O | $C_2H_5$ | Öl |
| 20 | 6-$CH_3$ O | $C_3H_7$ | 138 |
| 21 | 6-$CH_3$ O | $C_4H_9$ | |
| 22 | 6-$CH_3$ O | $HO\text{-}CH_2$ | 125 Zers. |
| 23 | 6-$CH_3$ O | $CH_3$ CH(OH)- | 156 |
| 24 | 6-$CH_3$ O | $CH_3$ O-$CH_2$ | 167 |

| Beispiel Nr. | $R^1_n$ | $R^2$ | Schmp. °C |
|---|---|---|---|
| 25 | 6-$C_2H_5$ O | $C_2H_5$ | |
| 26 | 6-$C_2H_5$ O | $C_3H_7$ | |
| 27 | 6-$C_2H_5$ O | $CH_3$ O-$CH_2$ | |
| 28 | 6-$C_3H_7$ O | $C_2H_5$ | |
| 28a | 6-OH | $CH_3SCH_2$ | 146 |

Beispiel 29:

R-4-Isopropoxycarbonyl-6-methoxy-3-(methylthiomethyl)-3,4-dihydrochinoxalin-2(1H)-on

11,9 g (0,05 mol) der Verbindung des Beispiels 3 werden in 300 ml Methylenchlorid unter Stickstoff suspendiert. Unter Rühren werden 7,0 g 4-Methylpyridin (0,075 mol) als Base rasch zugegeben. Anschließend werden 60 ml einer 1 molaren Lösung von Chlorameisensäureisopropylester in Toluol (0,06 mol) innerhalb von 30 Minuten bei Raumtemperatur zugetropft. Dabei geht die Suspension langsam in Lösung. Nach DC- Kontrolle ist die Reaktion nach 4 bis 6 Stunden bei Raumtemperatur beendet. Die Lösung wird mit 2n Schwefelsäure angesäuert, die organische Phase wird abgetrennt, die wässrige Phase noch einmal mit 50 ml Methylenchlorid extrahiert. Nach dem Abdampfen der Lösungsmittel unter vermindertem Druck verbleibt ein halbfestes Produkt, das unter Rühren aus Diisopropylether umkristallisiert wird.
Ausbeute: 15,0 g, Schmp. 115°C.
$^1$H-NMR (200 MHz, $d_6$-DMSO): $\delta$ = 1,3 (2d, J = 7 Hz, 6H, 2 Isopropyl-$CH_3$), 2,1 (s, 3 H, $SCH_3$), 2,35 + 2,7 ($dq_{AB}$, 2 H, -$CH_2$-S), 3,73 (s, 3 H, MeO), 4,87 (q, 1 H, CH), 4,97 (m, J = 7 Hz, 1 H, Isopropyl-CH), 6,7- 7,25 (m, 3 H, Aromaten), 10,65 (s, 1 H, Amid).
MS: Chemische Ionisation, $(M+H)^+$ = 325
HPLC: 98 % Reinheit, 99,9 % R-Enantiomer
Optische Rotation: $[\alpha]_D^{22}$ = 39° (c = 1 in Methanol)

| Analyse: | ber. | gef. |
|---|---|---|
| C | 55,6 % | 55,5 % |
| H | 6,2 % | 5,8 % |
| N | 8,6 % | 8,4 % |
| S | 9,8 % | 9,7 % |

In analoger Verfahrensweise zum Beispiel 29 können beispielsweise bei Verwendung von Verbindungen der Formel II, wie sie beispielsmäßig in den Beispielen 3 -28 genannt sind, durch Umsetzung mit den entsprechenden Verbindungen der Formel III, die folgenden Verbindungen der Formel I mit X = O erhalten werden:

Tabelle 2

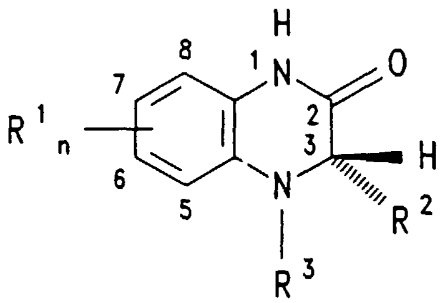

| Beispiel Nr. | $R^1_n$ | $R^2$ | $R^3$ | Schmp. °C |
|---|---|---|---|---|
| 30 | H (n = 0) | $C_2H_5$ | $COOCH(CH_3)_2$ | 163 |
| 31 | H (n = 0) | $C_3H_7$ | $COOCH(CH_3)_2$ | 117 |
| 32 | H (n = 0) | $C_4H_9$ | $COOCH(CH_3)_2$ | 120 |
| 33 | H (n = 0) | $HO-CH_2$ | $COOCH(CH_3)_2$ | |
| 34 | H (n = 0) | $CH_3SCH_2$ | $COOCH(CH_3)_2$ | 119 |
| 35 | 6-Cl | $C_2H_5$ | $COOCH(CH_3)_2$ | 145-147 |
| 36 | 6-Cl | $C_3H_7$ | $COOCH(CH_3)_2$ | |

| Beispiel Nr. | $R^1_n$ | $R^2$ | $R^3$ | Schmp. °C |
|---|---|---|---|---|
| 37 | 6-Cl | $C_4H_9$ | $COOCH(CH_3)_2$ | |
| 38 | 6-Cl | $CH_3SCH_2$ | $COOCH(CH_3)_2$ | 105 |
| 39 | 6-F | $C_2H_5$ | $COOCH(CH_3)_2$ | 123-125 |
| 40 | 6-F | $C_3H_7$ | $COOCH(CH_3)_2$ | 110 |
| 41 | 6-F | $C_4H_9$ | $COOCH(CH_3)_2$ | |
| 42 | 6-F | $CH_3SCH_2$ | $COOCH(CH_3)_2$ | 136 |
| 43 | 6-$CH_3$ O | $C_2H_5$ | $COOCH(CH_3)_2$ | Öl |
| 44 | 6-$CH_3$ O | $C_3H_7$ | $COOCH(CH_3)_2$ | 153 |
| 45 | 6-$CH_3$ O | $C_4H_9$ | $COOCH(CH_3)_2$ | |
| 46 | 6-$CH_3$ O | HO-$CH_2$ | $COOCH(CH_3)_2$ | Harz |
| 47 | 6-$CH_3$ O | $CH_3$ CH(OH)- | $COOCH(CH_3)_2$ | Harz |
| 48 | 6-$CH_3$ O | $CH_3$ O-$CH_2$ | $COOCH(CH_3)_2$ | 98 |
| 49 | 6-$C_2H_5$ O | $C_2H_5$ | $COOCH(CH_3)_2$ | |
| 50 | 6-$C_2H_5$ O | $C_3H_7$ | $COOCH(CH_3)_2$ | |
| 51 | 6-$C_2H_5$ O | $CH_3$ O-$CH_2$ | $COOCH(CH_3)_2$ | |
| 52 | 6-$C_2H_5$ O | $CH_3SCH_2$ | $COOCH(CH_3)_2$ | 112 |
| 53 | 6-$C_3H_7$ O | $C_2H_5$ | $COOCH(CH_3)_2$ | |
| 54 | 6-$C_3H_7$ O | $CH_3SCH_2$ | $COOCH(CH_3)_2$ | 105 |

| Beispiel Nr. | $R^1_n$ | $R^2$ | $R^3$ | Schmp. °C |
|---|---|---|---|---|
| 55 | H (n = 0) | $C_2H_5$ | $COOC(CH_3)=CH_2$ | |
| 56 | H (n = 0) | $CH_3SCH_2$ | $COOC(CH_3)=CH_2$ | |
| 57 | 6-Cl | $C_2H_5$ | $COOC(CH_3)=CH_2$ | 143 |
| 58 | 6-Cl | $C_2H_5$ | $COOCH_2CH=CH_2$ | 122-124 |
| 59 | 6-Cl | $CH_3SCH_2$ | $COOC(CH_3)=CH_2$ | 182 |
| 60 | 6-Cl | $CH_3SCH_2$ | $COOC_3H_7$ | 68 |
| 61 | 6-Cl | $CH_3SCH_2$ | $COOC_2H_5$ | 143 |
| 62 | 6-F | $C_2H_5$ | $COOC(CH_3)=CH_2$ | 125 |
| 63 | 6-F | $C_3H_7$ | $COOC(CH_3)=CH_2$ | |
| 64 | 6-F | $CH_3SCH_2$ | $COOC(CH_3)=CH_2$ | |
| 65 | 6-$CH_3$ O | $C_2H_5$ | $COOC(CH_3)=CH_2$ | |
| 66 | 6-$CH_3$ O | $C_3H_7$ | $COOC(CH_3)=CH_2$ | |
| 67 | 6-$CH_3$ O | $CH_3$ O-$CH_2$ | $COOC(CH_3)=CH_2$ | |
| 68 | 6-$CH_3$ O | $CH_3SCH_2$ | $COOC(CH_3)=CH_2$ | 152 |
| 70 | 6-$C_2H_5$ O | $C_2H_5$ | $COOC(CH_3)=CH_2$ | |
| 71 | 6-$C_2H_5$ O | $C_3H_7$ | $COOC(CH_3)=CH_2$ | |
| 72 | 6-$C_2H_5$ O | $CH_3$ O-$CH_2$ | $COOC(CH_3)=CH_2$ | |
| 73 | 6-$C_2H_5$ O | $CH_3SCH_2$ | $COOC(CH_3)=CH_2$ | |
| 74 | H (n = 0) | $C_2H_5$ | $COOC_2H_5$ | |
| 75 | H (n = 0) | $C_3H_7$ | $COOC_2H_5$ | |
| 76 | H (n = 0) | $CH_3SCH_2$ | $COOC_2H_5$ | |
| 77 | 6-Cl | $C_2H_5$ | $COOC_2H_5$ | |

| Beispiel Nr. | $R^1_n$ | $R^2$ | $R^3$ | Schmp. °C |
|---|---|---|---|---|
| 78 | 6-F | $C_2H_5$ | $COOC_2H_5$ | 116 |
| 79 | 6-F | $CH_3SCH_2$ | $COOC_2H_5$ | |
| 80 | 6-$CH_3$ O | $C_2H_5$ | $COOC_2H_5$ | |
| 81 | 6-$CH_3$ O | $CH_3$ O-$CH_2$ | $COOC_2H_5$ | |
| 82 | 6-$CH_3$ O | $CH_3SCH_2$ | $COOC_2H_5$ | |
| 83 | 6-$C_2H_5$ O | $C_2H_5$ | $COOC_2H_5$ | |
| 84 | 6-$C_2H_5$ O | $CH_3SCH_2$ | $COOC_2H_5$ | |
| 84a | 6-OH | $CH_3SCH_2$ | $COOCH(CH_3)_2$ | 182 |
| 84b | 6-OH | $C_2H_5$ | $COOCH(CH_3)_2$ | 201 |
| 84c | 6-Cl | $CH_3$ | $COOC_2H_5$ | 151 |
| 84d | 6-Cl | $C_4H_9$ | $COOC(CH_3)=CH_2$ | 158 |
| 84e | 6-Cl | $CH_3SCH_2$ | $COOC_2H_5$ | 143 |
| 84f | 6-Cl | $CH_3SCH_2$ | $COOC_3H_7$ | 68 |
| 84g | 6-$CH_3O$ | $CH_3SCH_2$ | $COOCH(CH_3)$-$C_2H_5$ | 86 |
| 84h | 6-$CH_3O$ | $CH_3SCH_2$ | $COOCH_2CH(CH_3)_2$ | 60 |
| 84i | 6-F | $CH_3$ | $COOCH(CH_3)_2$ | 151 |
| 84j | 6-F | $C_2H_5$ | $COOCH(CH_3)$-$C_2H_5$ | Harz |
| 84k | 6-F | $C_2H_5$ | $COOCH_3$ | 50 |
| 84l | 6-F | $C_2H_5$ | $COOC_4H_9$ | 92 |
| 84m | 6-F | $C_2H_5$ | $COOCH_2(CH_3)_2$ | 90 |
| 84n | 6-F | $CH_2OH$ | $COOCH(CH_3)_2$ | Harz |
| 84o | 6-F | $CH_3OCH_2$ | $COOCH(CH_3)_2$ | 114 |

Beispiel 85:

S-4-Isopropoxycarbonyl-6-methoxy-3-(methylthiomethyl)-3,4-dihydrochinoxalin-2(1H)-thion

16,1 g der Verbindung aus Beispiel 29 (0,05 mol) werden in200 ml trockenem Dimethoxyethan gelöst und unter Argon und Rühren mit 13 g fein gepulvertem Phosphorpentasulfid (0,06 mol) versetzt und bei Raumtemperatur gerührt. Nach 24 Stunden ist die Umsetzung noch nicht vollständig, so daß weitere 4 g Phosphorpentasulfid nachgegeben werden. Nach 24 bei Raumtemperatur wird noch 3 Stunden bei 30°C nachgerührt. Zur Abtrennung von Feststoffen wird über eine Klärschicht abgesaugt und mit Dimethoxyethan nachgewaschen. Die gesammelten Filtrate werden unter vermindertem Druck eingedampft. Das verbleibende dunkle Öl wird in 250 ml MTB-Ether aufgenommen und mit 200 ml gesättigter Natriumhydrogencarbonatlösung ausgerührt. Die Phasen werden getrennt, die wässrige Phase wird nochmals mit 20 ml MTB-Ether extrahiert. Die organischen Extrakte werden über Magnesiumsulfat oder Natriumsulfat getrocknet und einrotiert.

Das verbleibende gelbbraune Öl wird heiß in 30 ml Diisopropylether gelöst. Es kristallisiert beim Abkühlen unter Rühren aus. Die ausgefallenen Kristalle werden mit wenig Diisopropylether und n-Pentan gewaschen und im Exsiccator getrocknet.
Ausbeute 91,4 g, Schmp. 103°C,
$^1$H-NMR (200 MHz, $d_6$-DMSO): $\delta$ = 1,27 (2d, J = 7 Hz, 6H, 2 Isopropyl-$CH_3$), 2,1 (s, 3 H, $SCH_3$), 2,34 + 2,79 ($dq_{AB}$, 2 H, -$CH_2$-S), 3,75 (s, 3 H, MeO), 4,97 (m, J = 7 Hz, 1 H, Isopropyl-CH), 5,25 (q, 1 H, CH), 6,75 - 7,3 (m, 3 H, Aromaten), 12,73 (s, 1 H, Thioamid).
MS: Chemische Ionisation, $(M + H)^+$ = 341
HPLC: 99,6 % Reinheit, 99,4 % S-Enantiomer
Optische Rotation: $[\alpha]^{22}_{D}$ = 18° (c = 1 in Methanol)

| Analyse: | ber. | gef. |
|---|---|---|
| C | 52,9 % | 52,9 % |
| H | 5,9 % | 5,3 % |
| N | 8,4 % | 8,3 % |
| S | 18,8 % | 18,6 % |

In analoger Verfahrensweise zum Beispiel 85 können beispielsweise bei Verwendung von Verbindungen der Formel I mit X = O, wie sie beispielsmäßig in den Beispielen 30 bis 84 genannt sind, durch Umsetzung mit den entsprechenden Schwefelungsreagenzien, die folgenden Verbindungen der Formel I mit X = S erhalten werden:

Tabelle 3

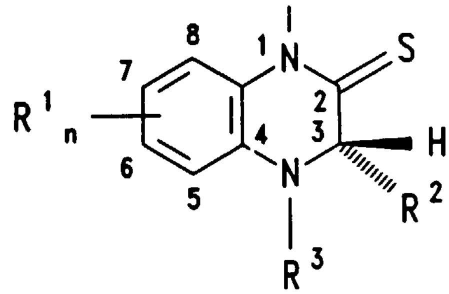

| Beispiel Nr. | $R^1_n$ | $R^2$ | $R^3$ | Schmp. °C |
|---|---|---|---|---|
| 86 | H (n = 0) | $C_2H_5$ | $COOCH(CH_3)_2$ | 114 |
| 87 | H (n = 0) | $C_3H_7$ | $COOCH(CH_3)_2$ | 128 |
| 88 | H (n = 0) | $C_4H_9$ | $COOCH(CH_3)_2$ | 78 |
| 89 | H (n = 0) | HO-$CH_2$ | $COOCH(CH_3)_2$ | |
| 90 | H (n = 0) | $CH_3SCH_2$ | $COOCH(CH_3)_2$ | Öl |
| 91 | 6-Cl | $C_2H_5$ | $COOCH(CH_3)_2$ | 161 |
| 92 | 6-Cl | $C_3H_7$ | $COOCH(CH_3)_2$ | |
| 93 | 6-Cl | $C_4H_9$ | $COOCH(CH_3)_2$ | |
| 94 | 6-Cl | $CH_3SCH_2$ | $COOCH(CH_3)_2$ | 124 |
| 95 | 6-F | $C_2H_5$ | $COOCH(CH_3)_2$ | 93 |

| Beispiel Nr. | $R^1_n$ | $R^2$ | $R^3$ | Schmp. °C |
|---|---|---|---|---|
| 96 | 6-F | $C_3H_7$ | $COOCH(CH_3)_2$ | 60 |
| 97 | 6-F | $C_4H_9$ | $COOCH(CH_3)_2$ | |
| 98 | 6-F | $CH_3SCH_2$ | $COOCH(CH_3)_2$ | 122 |
| 99 | 6-$CH_3$ O | $C_2H_5$ | $COOCH(CH_3)_2$ | 74 |
| 100 | 6-$CH_3$ O | $C_3H_7$ | $COOCH(CH_3)_2$ | 140 |
| 101 | 6-$CH_3$ O | $C_4H_9$ | $COOCH(CH_3)_2$ | |
| 102 | 6-$CH_3$ O | HO-$CH_2$ | $COOCH(CH_3)_2$ | |
| 103 | 6-$CH_3$ O | $CH_3$ CH(OH)- | $COOCH(CH_3)_2$ | |
| 104 | 6-$CH_3$ O | $CH_3$ O-$CH_2$ | $COOCH(CH_3)_2$ | 137 |
| 105 | 6-$C_2H_5$ O | $C_2H_5$ | $COOCH(CH_3)_2$ | |
| 106 | 6-$C_2H_5$ O | $C_3H_7$ | $COOCH(CH_3)_2$ | |
| 107 | 6-$C_2H_5$ O | $CH_3$ O-$CH_2$ | $COOCH(CH_3)_2$ | |
| 108 | 6-$C_2H_5$ O | $CH_3SCH_2$ | $COOCH(CH_3)_2$ | Öl |
| 109 | 6-$C_3H_7$ O | $C_2H_5$ | $COOCH(CH_3)_2$ | |
| 110 | 6-$C_3H_7$ O | $CH_3SCH_2$ | $COOCH(CH_3)_2$ | Harz |

| Beispiel Nr. | $R^1_n$ | $R^2$ | $R^3$ | Schmp. °C |
|---|---|---|---|---|
| 111 | H (n = 0) | $C_2H_5$ | $COOC(CH_3)=CH_2$ | |
| 112 | H (n = 0) | $CH_3SCH_2$ | $COOC(CH_3)=CH_2$ | |
| 113 | 6-Cl | $C_2H_5$ | $COOC(CH_3)=CH_2$ | 170 |
| 114 | 6-Cl | $C_2H_5$ | $COOCH_2CH=CH_2$ | 123 |

| Beispiel Nr. | $R^1_n$ | $R^2$ | $R^3$ | Schmp. °C |
|---|---|---|---|---|
| 115 | 6-Cl | $CH_3SCH_2$ | $COOC(CH_3)=CH_2$ | 128 |
| 116 | 6-Cl | $CH_3SCH_2$ | $COOC_3H_7$ | |
| 117 | 6-Cl | $CH_3SCH_2$ | $COOC_2H_5$ | |
| 118 | 6-F | $C_2H_5$ | $COOC(CH_3)=CH_2$ | |
| 119 | 6-F | $C_3H_7$ | $COOC(CH_3)=CH_2$ | |
| 120 | 6-F | $CH_3SCH_2$ | $COOC(CH_3)=CH_2$ | |
| 121 | 6-$CH_3$ O | $C_2H_5$ | $COOC(CH_3)=CH_2$ | |
| 122 | 6-$CH_3$ O | $C_3H_7$ | $COOC(CH_3)=CH_2$ | |
| 123 | 6-$CH_3$ O | $CH_3$ O-$CH_2$ | $COOC(CH_3)=CH_2$ | |
| 124 | 6-$CH_3$ O | $CH_3SCH_2$ | $COOC(CH_3)=CH_2$ | 152 |
| 126 | 6-$C_2H_5$ O | $C_2H_5$ | $COOC(CH_3)=CH_2$ | |
| 127 | 6-$C_2H_5$ O | $C_3H_7$ | $COOC(CH_3)=CH_2$ | |
| 128 | 6-$C_2H_5$ O | $CH_3$ O-$CH_2$ | $COOC(CH_3)=CH_2$ | |
| 129 | 6-$C_2H_5$ O | $CH_3SCH_2$ | $COOC(CH_3)=CH_2$ | |
| 130 | H (n = 0) | $C_2H_5$ | $COOC_2H_5$ | |
| 131 | H (n = 0) | $C_3H_7$ | $COOC_2H_5$ | |
| 132 | H (n = 0) | $CH_3SCH_2$ | $COOC_2H_5$ | |
| 133 | 6-Cl | $C_2H_5$ | $COOC_2H_5$ | |
| 134 | 6-F | $C_2H_5$ | $COOC_2H_5$ | Harz |
| 135 | 6-F | $CH_3SCH_2$ | $COOC_2H_5$ | |
| 136 | 6-$CH_3$ O | $C_2H_5$ | $COOC_2H_5$ | |
| 137 | 6-$CH_3$ O | $CH_3$ O-$CH_2$ | $COOC_2H_5$ | |

| Beispiel Nr. | $R^1_n$ | $R^2$ | $R^3$ | Schmp. °C |
|---|---|---|---|---|
| 138 | 6-$CH_3$ O | $CH_3SCH_2$ | $COOC_2H_5$ | |
| 139 | 6-$C_2H_5$ O | $C_2H_5$ | $COOC_2H_5$ | |
| 140 | 6-$C_2H_5$ O | $CH_3SCH_2$ | $COOC_2H_5$ | |
| 140a | 6-OH | $CH_3SCH_2$ | $COOCH(CH_3)_2$ | 113 |
| 140b | 6-OH | $C_2H_5$ | $COOCH(CH_3)_2$ | Harz |
| 140c | 6-Cl | $CH_3$ | $COOCH_2CH=CH_2$ | 144 |
| 140d | 6-Cl | $CH_3$ | $COOC(CH_3)=CH_2$ | 149 |
| 140e | 6-Cl | $C_4H_9$ | $COOC(CH_3)=CH_2$ | 132 |
| 140f | 6-$CH_3O$ | $CH_3SCH_2$ | $COOCH(CH_3)$-$C_2H_5$ | 60 |
| 140g | 6-$CH_3O$ | $CH_3SCH_2$ | $COOCH_2CH(CH_3)_2$ | 89 |
| 140h | 6-F | $C_2H_5$ | $COOCH_3$ | 146 |
| 140i | 6-F | $C_2H_5$ | $COOC_4H_9$ | 103 |
| 140j | 6-F | $C_2H_5$ | $COOCH_2CH(CH_3)_2$ | Harz |
| 140k | 6-F | $C_2H_5$ | $COOCH(CH_3)$-$C_2H_5$ | 51 |
| 140l | 6-F | $CH_3OCH_2$ | $COOCH(CH_3)_2$ | 143 |

Wirksamkeitstests:
Prüfung von Präparaten gegen HIV in der Zellkultur
Methodenbeschreibung:
Medium: RMPI, pH 6,8
Komplettes Medium enthält zusätzlich 20 % foetales Kälberserum und 40 IU/ml rekombinantes Interleukin 2.

Zellen:

Aus frischem Spenderblut mittels Ficoll[R]-Gradienten- Zentrifugation isolierte Lymphozyten werden unter Zusatz von 2 g/ml Phytohämagglutinin (Wellcome) in komplettem Medium 36 Stunden bei 37°C unter 5 % $CO_2$ kultiviert. Die Zellen werden nach Zusatz von 10 % DMSO bei einer Zelldichte von 5 x $10^6$ eingefroren und in flüssigem Stickstoff gelagert. Für den Versuch werden die Zellen aufgetaut, im RPMI-Medium gewaschen und im kompletten Medium 3 bis 4 Tage kultiviert.

Ansatz:

Die Prüfpräparate wurden in einer Konzentration von 16,7 mg/ml in DMSO gelöst und in komplettem Medium auf 1 mg/ml verdünnt.

In 24er Multiwell-Schalen wurden 0,4 ml Medium vorgelegt. Nach Zugabe von 0,1 ml des gelösten Präparates in die obere Reihe der Schale wurde durch Übertragung von jeweils 0,1 ml eine geometrische Verdünnungsreihe erzeugt. Präparatfreie Kontrollen enthielten stets 0,4 ml komplettes Medium mit 0,5 % DMSO.
Lymphozytenkulturen mit einer Zellzahl von 5 x $10^5$ Zellen/ml wurden durch Zugabe 1/50 Volumen Überstand aus HIV-infizierten Lymphozytenkulturen infiziert. Der Titer dieser Kulturüberstände wurde durch Endpunktverdünnung mit 1 - 5 x $10^6$ infektiöse Einheiten/ml bestimmt. Nach 30 min. Inkubation bei 37°C wurden die infizierten Lymphozyten abzentrifugiert und im gleichen Volumen Medium wieder aufgenommen. Von dieser Zellsuspension wurden jeweils 0,6 ml in alle Vertiefungen der Testplatte gegeben. Die Ansätze wurden 3 Tage bei 37°C inkubiert.

Auswertung:

Die infizierten Zellkulturen wurden unter dem Mikroskop auf Anwesenheit von Riesenzellen untersucht, die eine aktive Virusvermehrung in der Kultur anzeigen. Die geringste Präparatekonzentration, bei der keine Riesenzellen auftraten, wurde als Hemmkonzentration gegen HIV bestimmt. Zur Kontrolle wurden die Überstände aus den Kulturplatten mit Hilfe eines HIV- Antigentests entsprechend den Angaben des Herstellers (Organon) auf Anwesenheit von HIV-Antigen bestimmt.

Ergebnisse:

Tabelle 4:

| Verbindung von Beispiel Nr. | T-Zellkulturassay MHK $EC_{50}$ (ng/ml) |
|---|---|
| 29 | <8 |
| 30 | <40 |
| 31 | 50 |
| 34 | <1 |
| 35 | <80 |
| 38 | <1 |
| 42 | <8 |
| 43 | <1 |
| 44 | <80 |
| 52 | <8 |
| 54 | 40 |
| 57 | 1 |
| 58 | 10 |
| 59 | 20 |
| 60 | 40 |
| 61 | 2 |
| 68 | 8 |
| 85 | 2 |
| 86 | 1 |
| 87 | 4 |
| 88 | <40 |
| 90 | <8 |
| 91 | 2 |

Tabelle 4: (fortgesetzt)

| Verbindung von Beispiel Nr. | T-Zellkulturassay MHK $EC_{50}$ (ng/ml) |
|---|---|
| 94 | <8 |
| 95 | 2 |
| 99 | <1 |
| 100 | 4 |
| 108 | <5 |
| 110 | 4 |
| 113 | 0,8 |
| 114 | 1,6 |
| 115 | 1,6 |
| 124 | <8 |
| 39 | 8 |
| 40 | 80 |
| 62 | 80 |
| 78 | <80 |
| 84j | 80 |
| 84j | <80 |
| 84l | 80 |
| 840 | 80 |
| 96 | 80 |
| 98 | 3 |
| 104 | 8 |
| 134 | 8 |
| 140a | 40 |
| 140b | <80 |
| 140c | 40 |
| 140d | 10 |
| 140f | 8 |
| 140g | 40 |
| 140h | 10 |
| 140i | 10 |
| 140j | 10 |
| 140k | 8 |
| 140l | 8 |

Untersuchung der Substanzen auf Hemmung der HIV-"Reverse Transkriptase"

Die Aktivität der Reversen Transkriptase (RT) wurde mit Hilfe eines "Scintillation Proximity Assay" (SPA) bestimmt.

Das Reagenzkit für den RT-SPA wurde von Amersham/Buchler (Braunschweig) bezogen. Das Enzym RT (aus HIV in E. coli cloniert) stammte von der Firma HT-Biotechnology Ltd, Cambridge, UK.

Ansatz:

Der Test wurde nach dem Methoden-Manual des Herstellers Amersham durchgeführt - mit folgenden Modifikationen:

- dem "Assay"-Puffer wurde Rinderserumalbumin zu der Endkonzentration 0,5 mg/ml zugesetzt.
- der Test wurde in Eppendorf-Reaktionsgefäßen mit 100 µl Ansatzvolumen durchgeführt.
- das RT-Konzentrat des Herstellers (5000 U/ml) wurde in Tris-HCl Puffer 20 mM; pH 7,2; 30 % Glycerin auf eine Aktivität von 15 U pro ml verdünnt.
- die Inkubationszeit für die Ansätze betrug 60 min (37°C).
- nach Abstoppen der Reaktion und "Entwicklung" mit der Perlen-Suspension wurden 130 µl Ansatz in 4,5 ml Tris-HCl Puffer, 10 mM; pH 7,4; 0,15 M NaCl transferiert und die Tritium-Aktivität in einem β-Counter gemessen.

Substanzprüfung

Für eine Vorprüfung der Inhibitoraktivität wurden die Substanzen in DMSO gelöst (Stammlösung c = 1 mg/ml) und in Verdünnung in DMSO $10^{-1}$, $10^{-2}$, $10^{-3}$ usw. getestet.
Zur Bestimmung von $IC_{50}$-Werten wurden die Inhibitor-Stammlösungen in Tris-HCl Puffer, 50 mM, pH 8 weiterverdünnt und in geeigneten Konzentrationen getestet.
Aus der graphischen Darstellung RT-Aktivität versus log $C_{Inh.}$ wurde die einer 50 %igen Enzymhemmung zugehörige Konzentration ermittelt.
Die Ergebnisse der Untersuchung zeigt Tabelle 5.

Tabelle 5

| Verbindung von Beispiel Nr. | Reverse Transkriptase Assay $IC_{50}$ (ng/ml) |
|---|---|
| 29 | 10-100 |
| 34 | 10-100 |
| 35 | 10 |
| 38 | 5 |
| 52 | 10-100 |
| 57 | 10-100 |
| 58 | 10-100 |
| 59 | 18 |
| 60 | 10 |
| 61 | 10-100 |
| 68 | 16 |
| 85 | 8 |
| 86 | 11 |
| 87 | 27 |
| 90 | 5 |
| 91 | 4 |
| 94 | 15 |
| 99 | 11 |
| 100 | 16 |
| 108 | 8 |
| 110 | 10-100 |
| 113 | 6 |

Tabelle 5 (fortgesetzt)

| Verbindung von Beispiel Nr. | Reverse Transkriptase Assay $IC_{50}$ (ng/ml) |
|---|---|
| 114 | 7 |
| 115 | 10 |
| 125 | 15 |
| 39 | 20 |
| 40 | 10-100 |
| 62 | 92 |
| 78 | 80 |
| 84g | 118 |
| 84i | 170 |
| 84j | 87 |
| 84l | 150 |
| 96 | 16 |
| 98 | 12 |
| 104 | 35 |
| 134 | 3 |
| 140a | 93 |
| 140b | 70 |
| 140c | 110 |
| 140d | 27 |
| 140f | 19 |
| 140g | 17 |
| 140h | 8 |
| 140i | 22 |
| 140j | 15 |
| 140k | 16 |
| 140l | 22 |

**Patentansprüche**

**1.** Verbindungen der Formel I und Ia,

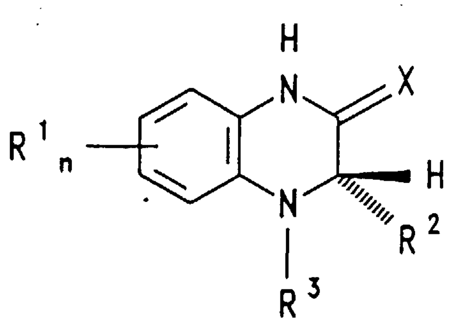

(I)

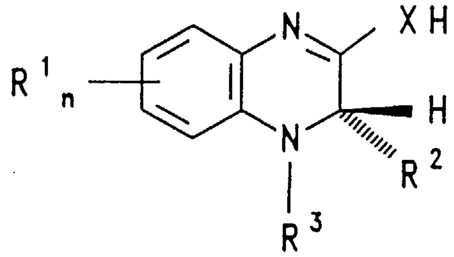

(I a)

sowie deren physiologisch verträgliche Salze und Prodrugs, wobei in den Formeln I und Ia bedeuten:

n gleich null oder eins,

$R^1$ Fluor, Chlor, Hydroxy, $C_1$-$C_3$-Alkoxy,

$R^2$ $C_1$-$C_4$-Alkyl, gegebenenfalls substituiert durch Hydroxy, $C_1$-$C_4$-Alkoxy, oder $C_1$-$C_4$-Alkylthio,

$R^3$ $C_1$-$C_4$-Alkyloxycarbonyl oder $C_2$-$C_4$-Alkenyloxycarbonyl und

X bedeutet Sauerstoff oder Schwefel.

**2.** Verbindungen der Formel I bzw. Ia gemäß Anspruch 1 dadurch gekennzeichnet, daß die Substituenten in den genannten Formeln bedeuten:

n gleich null oder eins,

$R^1$ Fluor, Chlor, Methoxy, Ethoxy, Propoxy,

$R^2$ Methylthiomethyl, Ethyl, Propyl, $C_1$-$C_4$-Alkyl, substituiert durch Hydroxy oder $C_1$-$C_4$-Alkoxy,

$R^3$ $C_1$-$C_4$-Alkyloxycarbonyl oder $C_2$-$C_4$-Alkenyloxycarbonyl,

X bedeutet Sauerstoff oder Schwefel.

**3.** Verbindungen der Formel I oder Ia gemäß den Ansprüchen 1 bis 2, dadurch gekennzeichnet, daß die Substituenten in den genannten Formeln bedeuten:

n gleich null oder eins,

$R^1$ Fluor, Chlor, Methoxy, Ethoxy,

$R^2$ Methylthiomethyl, Ethyl, Propyl, $C_1$-$C_2$-Alkyl substituiert durch Hydroxy oder $C_1$-$C_4$-Alkoxy,

$R^3$ $C_1$-$C_4$-Alkyloxycarbonyl oder $C_2$-$C_4$-Alkenyloxycarbonyl,

X bedeutet Sauerstoff oder Schwefel.

**4.** Verfahren zur Herstellung von Verbindungen der Formel I bzw. Ia gemäß Anspruch 1, dadurch gekennzeichnet, daß man

A) zur Herstellung von Verbindungen der Formel I mit X gleich Sauerstoff und den Resten $R^1$, $R^2$ und $R^3$ wie in den Ansprüchen 1 bis 4 definiert eine Verbindung der Formel II,

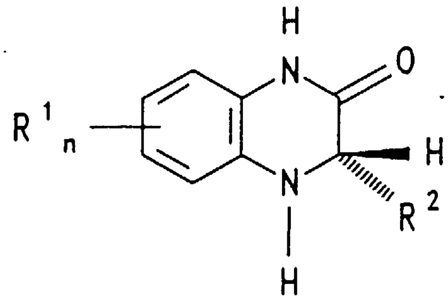

(II)

wobei für $R^1$ und $R^2$ die in den Ansprüchen 1 bis 3 genannten Definitionen gelten, mit einer Verbindung der Formel III,

$$R^3\text{-}Z \qquad \text{(III)}$$

wobei $R^3$ die in den Ansprüchen 1 bis 3 genannten Bedeutungen hat und Z eine Abgangsgruppe wie zum Beispiel Chlor ist, umsetzt oder daß man

B) Verbindungen der Formel I, mit X gleich Schwefel und $R^1$, $R^2$ und $R^3$ wie in den Ansprüchen 1 bis 3 definiert herstellt durch Reaktion einer Verbindung der Formel I, wobei X Sauerstoff ist und für $R^1$, $R^2$ und $R^3$ die in den Ansprüchen 1 bis 3 genannten Definitionen gelten, mit einem Schwefelungsreagenz.

**5.** Arzneimittel, enthaltend eine wirksame Menge mindestens einer Verbindung der Formel I bzw. Ia gemäß einem oder mehreren der Ansprüche 1 bis 3.

**6.** Verfahren zur Herstellung eines Arzneimittels gemäß Anspruch 5, dadurch gekennzeichnet, daß die wirksame Menge einer Verbindung der Formel I bzw. Ia mit üblichen pharmazeutischen Hilfsstoffen in eine geeignete Darreichungsform gebracht wird.

**7.** Verwendung von Verbindungen der Formel I bzw. Ia gemäß einem oder mehreren der Ansprüche 1 bis 3 zur Herstellung von Arzneimitteln zur Behandlung von Viruserkrankungen.

**8.** Verbindungen gemäß einem oder mehreren der Ansprüche 1 bis 3 zur Anwendung als Arzneimittel.

## Claims

**1.** A compound of the formula I or Ia,

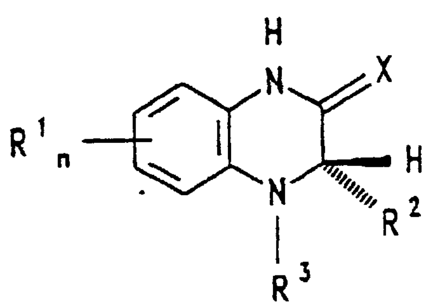

(I)

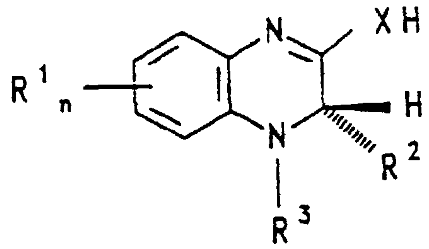

(Ia)

and the physiologically tolerated salts and prodrugs thereof, wherein, in formulae I and Ia:

n is zero or one,

$R^1$ is fluorine, chlorine, hydroxyl or $C_1$-$C_3$-alkoxy,

$R^2$ is $C_1$-$C_4$-alkyl which is optionally substituted by hydroxyl, $C_1$-$C_4$-alkoxy or $C_1$-$C_4$-alkylthio,

$R^3$ is $C_1$-$C_4$-alkyloxycarbonyl or $C_2$-$C_4$-alkenyloxycarbonyl, and

X is oxygen or sulfur.

**2.** A compound of the formula I or Ia as claimed in claim 1, wherein the substituents in the said formulae have the following meanings:

n is zero or one,

$R^1$ is fluorine, chlorine, methoxy, ethoxy or propoxy,

$R^2$ is methylthiomethyl, ethyl or propyl, or $C_1$-$C_2$-alkyl which is substituted by hydroxyl or $C_1$-$C_4$-alkoxy,

$R^3$ is $C_1$-$C_4$-alkyloxycarbonyl or $C_2$-$C_4$-alkenyloxycarbonyl,

X is oxygen or sulfur.

**3.** A compound of the formula I or Ia as claimed in claims 1 to 2, wherein the substituents in the said formulae have the following meanings:

n is zero or one,

$R^1$ is fluorine, chlorine, methoxy or ethoxy,

$R^2$ is methylthiomethyl, ethyl or propyl, or $C_1$-$C_2$-alkyl which is substituted by hydroxyl or $C_1$-$C_4$-alkoxy,

$R^3$ is $C_1$-$C_4$-alkyloxycarbonyl or $C_2$-$C_4$-alkenyloxycarbonyl,

X is oxygen or sulfur.

**4.** A process for preparing compounds of the formula I or Ia as claimed in claim 1, which comprises

A) for preparing compounds of the formula I in which X is oxygen and the radicals $R^1$, $R^2$ and $R^3$ are defined as in claims 1 to 4, reacting a compound of the formula II,

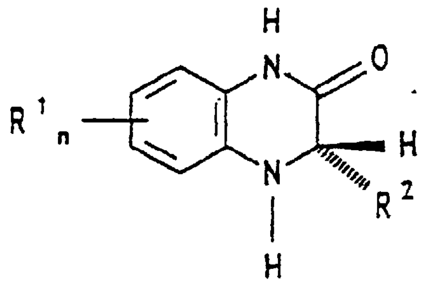

(II)

where the definitions mentioned in claims 1 to 3 apply to $R^1$ and $R^2$, with a compound of the formula III,

$$R^3\text{-}Z \qquad \text{(III)}$$

where $R^3$ has the meanings mentioned in claims 1 to 3 and Z is a leaving group such as, for example, chlorine, or which comprises

B) preparing compounds of the formula I, in which X is sulfur and $R^1$, $R^2$ and $R^3$ are defined as in claims 1 to 3, by reacting a compound of the formula I, where X is oxygen and the definitions mentioned in claims 1 to 3 apply to $R^1$, $R^2$ and $R^3$, with a sulfurization reagent.

**5.** A pharmaceutical which contains an effective quantity of at least one compound of the formula I or Ia as claimed in one or more of claims 1 to 3.

**6.** A process for preparing a pharmaceutical as claimed in claim 5, which comprises bringing the effective quantity of a compound of the formula I or Ia, together with customary pharmaceutical auxiliary substances, into a suitable form for administration.

**7.** The use of compounds of the formula I or Ia as claimed in one or more of claims 1 to 3 for preparing pharmaceuticals for treating viral diseases.

**8.** A compound as claimed in one or more of claims 1 to 3 for use as a pharmaceutical.

**Revendications**

**1.** Composés de formules I et Ia

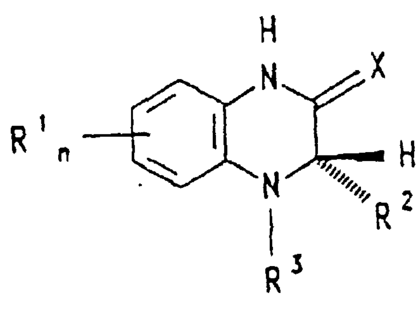

(I)

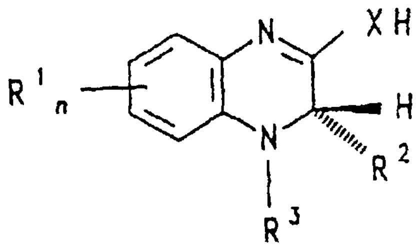

(Ia)

ainsi que leurs sels physiologiquement acceptables et précurseurs, les symboles dans les formules I et Ia ayant les significations suivantes :

- n est égal à zéro ou un,
- $R^1$ représente un atome de fluor ou de chlore ou un groupe hydroxy ou alcoxy en $C_1$-$C_3$,
- $R^2$ représente un groupe alkyle en $C_1$-$C_4$, éventuellement substitué par un groupe hydroxy, alcoxy en $C_1$-$C_4$ ou alkyl($C_1$-$C_4$)thio,
- $R^3$ représente un groupe alkyloxy($C_1$-$C_4$)carbonyle ou alcényloxy($C_2$-$C_4$)-carbonyle,
- X représente un atome d'oxygène ou de soufre.

**2.** Composés de formule I ou Ia selon la revendication 1, caractérisés en ce que les symboles dans les formules indiquées ont les significations suivantes :

- n est égal à zéro ou un,
- $R^1$ représente un atome de fluor ou de chlore ou le groupe méthoxy, éthoxy ou propoxy,
- $R^2$ représente le groupe méthylthiométhyle, éthyle ou propyle, ou un groupe alkyle en $C_1$-$C_2$ substitué par un groupe hydroxy ou alcoxy en $C_1$-$C_4$,
- $R^3$ représente un groupe alkyloxy($C_1$-$C_4$)carbonyle ou alcényloxy($C_2$-$C_4$)-carbonyle,
- X représente un atome d'oxygène ou de soufre.

**3.** Composés de formule I ou Ia selon les revendications 1 et 2, caractérisés en ce que les symboles dans les formules indiquées ont les significations suivantes :

- n est égal à zéro ou un,
- $R^1$ représente un atome de fluor ou de chlore ou le groupe methoxy ou éthoxy,
- $R^2$ représente le groupe méthylthiométhyle, éthyle ou propyle, ou un groupe alkyle en $C_1$-$C_2$ substitué par un groupe hydroxy ou alcoxy en $C_1$-$C_4$,
- $R^3$ représente un groupe alkyloxy($C_1$-$C_4$)carbonyle ou alcényloxy($C_2$-$C_4$)-carbonyle,
- X représente un atome d'oxygène ou de soufre.

**4.** Procédé pour la préparation de composés de formule I ou Ia selon la revendication 1, caractérisé en ce que

A) pour la préparation des composés de formule I dans lesquels X représente un atome d'oxygène et les radicaux $R^1$, $R^2$ et $R^3$ sont tels que définis dans les revendications 1 à 3, on fait réagir un composé de formule II

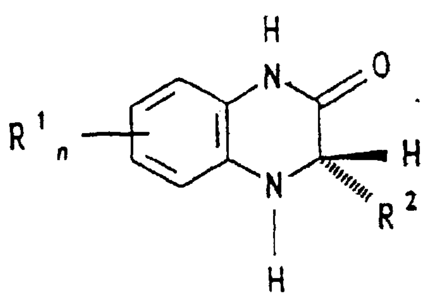

(II)

dans laquelle les définitions données dans les revendications 1 à 3 pour $R^1$ et $R^2$ sont valables, avec un

composé de formule III

$$R^3\text{-}Z \qquad (III)$$

dans laquelle $R^3$ a les significations données dans les revendications 1 à 3, et Z est un groupe partant, comme par exemple un atome de chlore, ou en ce que
B) on prépare des composés de formule I dans lesquels X est un atome de soufre et $R^1$, $R^2$ et $R^3$ sont tels que définis dans les revendications 1 à 3, en faisant réagir avec un réactif de sulfuration un composé de formule I, dans lequel X représente un atome d'oxygène et les définitions données pour $R^1$, $R^2$ et $R^3$ dans les revendications 1 à 3 sont valables.

**5.** Médicament, contenant une quantité efficace d'au moins un composé de formule I ou Ia selon une ou plusieurs des revendications 1 à 3.

**6.** Procédé pour la fabrication d'un médicament selon la revendication 5, caractérisé en ce que l'on met sous une forme d'administration appropriée la quantité efficace d'un composé de formule I ou la, avec des adjuvants pharmaceutiques usuels.

**7.** Utilisation des composés de formule I ou la selon une ou plusieurs des revendications 1 à 3, pour la fabrication d'un médicament destiné au traitement de maladies virales.

**8.** Composés selon une ou plusieurs des revendications 1 à 3, pour utilisation en tant que médicament.